# EUROPEAN PATENT APPLICATION

(11) **EP 2 090 997 A1**
(43) Date of publication of application: **19.08.2009**
(21) Application number: 08250485.3
(22) Date of filing: 08.02.2008
(51) Int. Cl.: G06F 19/00

(54) **Conveying real time medical data**

(71) Applicant: ISEEU Global Limited, Arlington Square Downshire Way Bracknell, Berkshire RG12 1WA (GB)
(72) Inventor: Donoghue Tony John, Crowthorne Berkshire RG45 7HP (GB); Clements Stephen, Eton-Wick Berkshire SL4 6LG (GB); Al-Shaikh Loua Hanna, Winchester Hampshire SO22 5FQ (GB)
(74) Representative: Atkinson, Ralph

(57) **Abstract**

There is provided apparatus for conveying real-time medical data received from patient connected terminals within a hospital to clinician. The apparatus is **characterised in that** the clinicians receive data from the apparatus via mobile devices (117) located outside a hospital and the apparatus is located at the hospital collecting a plurality of data sets from a plurality of patients. The apparatus comprises a first processing system (201) for receiving native medical data from a hospital internal network and for converting the data into generic video images; a second processing system (202) for authenticating requests from mobile devices to facilitate or block external communication; and a third processing system (203) for receiving input commands from the mobile devices, encrypting the generic figure images to produce encrypted video images and for transmitting the encrypted video images to the mobile devices.

## Description

### CROSS REFERENCE TO RELATED APPLICATIONS

This application represents the first application for a patent directed toward the invention and the subject matter.

### BACKGROUND OF THE INVENTION

### 1. Field of the Invention

The present invention relates to apparatus for conveying real-time medical data received from patient-connected terminals within a hospital to a clinician.

### 2. Description of the Related Art

In the field of medicine, there are many measurements taken from patients with the monitoring of these measurements being done typically at the patient's bedside. In addition to continuous trace measurements such as heart rate and blood pressure etc there are also snap shot type measurements taken such as x-rays and scans etc along with historical records such as pharmacological records.

Often, clinicians are required to review measurements in order to assess what treatment is required, to determine how a patient is progressing generally and possibly in order to respond to emergency conditions. Highly qualified clinicians are required to oversee a large number of cases and increasingly it is possible that these cases will be geographically spread thereby allowing clinicians to concentrate on particular medical areas. As a result, considerable travelling may be required or courier charges may be incurred if data is to be transferred manually between these organisations. Thus, if a clinician is not close at hand when an emergency situation arises, considerable time may be taken in order to reach a diagnosis and clearly under such circumstances this delay may be life critical.

### BRIEF SUMMARY OF THE INVENTION

According to an aspect of the present invention, there is provided apparatus of the aforesaid type, characterised by a first processing system for receiving native medical data from a hospital internal network and for converting said data into generic video images;
a second processing system for authenticating a request from a mobile device to facilitate or block external communication; and
a third processing system for receiving input commands from said mobile device, encrypting said generic video images to produce encrypted video images and for transmitting said encrypted video images.

### BRIEF DESCRIPTION OF THE SEVERAL VIEWS OF THE DRAWINGS

Figure 1 shows a hospital ward that includes medical devices and a data processing system;
Figure 2 shows a schematic representation of the functionality of the data processing system shown in Figure 1;
Figure 3 shows an example of components contained within data processing system 114;
Figure 4 shows the functionality of core processing environment 300;
Figure 5 shows an example of a display shown in order to add a clinician;
Figure 6 shows a mobile device wherein the remote monitoring facility is being installed;
Figure 7 shows an overview of procedures when a request is received from a clinician to receive data;
Figure 8 shows an example of a display shown requesting login details;
Figure 9 shows an example of an alternative channel for receipt of a one-time password;
Figure 10 shows a display provided to allow a clinician to select patients;
Figure 11 shows a display provided to allow a clinician to select medical monitoring devices;
Figure 12 shows interactions between the mobile device and processing system;
Figure 13 shows further detail of a request before data being processed; and
Figure 14 shows an example of medical data being displayed.

### DESCRIPTION OF THE BEST MODE FOR CARRYING OUT THE INVENTION

### Figure 1

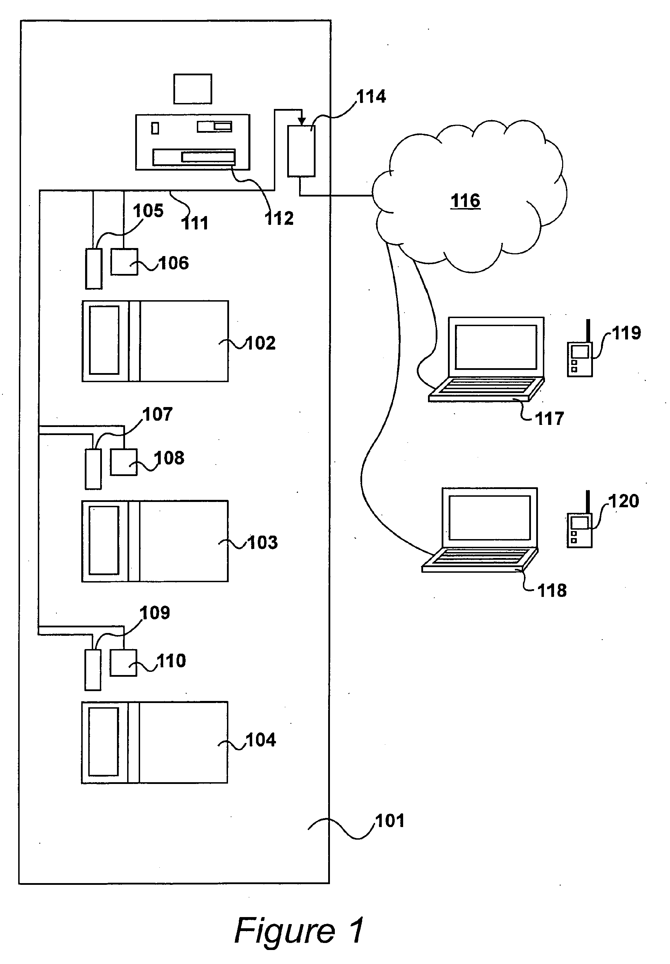

A hospital ward 101 is shown in Figure 1 that may be considered as a high dependency intensive care unit. A number of beds 102, 103, 104 are provided so that a plurality of patients may be accommodated. The beds are provided with medical devices, specifically devices 105 and 106 at bed 102, devices 107 and 108 at bed 103 and devices 109 and 110 at bed 104. The specific devices chosen for each particular patient will depend upon the patient's condition. However, they will typically include heart rate monitors, blood pressure monitors, blood oxygen level monitors, fluid flow monitors including devices for administering drugs, and imaging devices. These devices could take measurements such as ongoing trace type measurements such as heart trace etc or they could take snap-shot type measurements. Thus, the medical devices take measurements from a patient and produce input signals for local display. In addition, in this embodiment, each device includes an electronic network interface for networking the device to a local area network 111.

Data received on local area network 111 may be viewed at a local station 112. In addition, the environment is provided with a data processing system 114 that is configured to convey real-time medical data received from the patient-connected terminals within the hospital ward to mobile devices operated by clinicians outside the hospital. The data processing system 114 is connected to a public network 116 and a first mobile device 117 is connected to public network 116, and a second mobile device 118 is connected to public network 116 in this embodiment. In addition, the clinician using mobile device 117 is provided with a first mobile cellular telephone 119 and a clinician using mobile device 118 is provided with a second mobile cellular telephone 120; each of said telephones being enabled for receiving text (SMS) messages.

### Figure 2

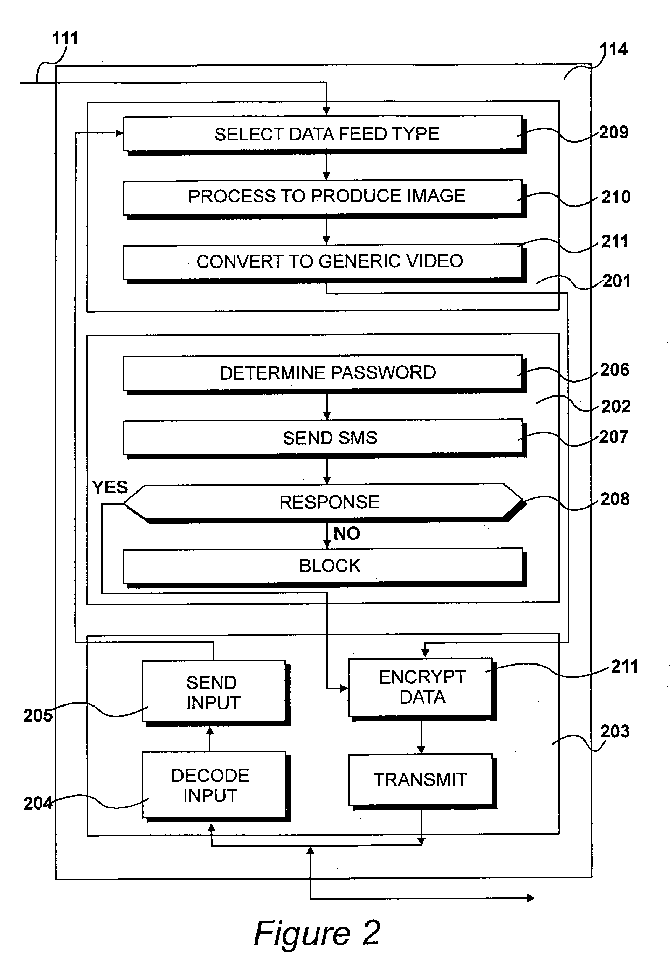

A schematic representation of the functionality provided by the data processing system 114 is shown in Figure 2. The data processing system 114 includes a first processing system 201 that is configured to receive native medical data from the hospital internal network 111 and to convert this into generic video images.

A second processing system 202 is configured to authenticate a request from a mobile device so as to facilitate or block external communications. A third processing system 203 is configured to receive input commands from the mobile devices, encrypt the generic video images to produce encrypted video images and to transmit these encrypted video images.

In order to describe the operations performed within the environment of Figure 2, by way of example only, it is assumed that a clinician mobile device 117 receives real-time data from monitor 106, monitoring a patient in bed 102, at a remote location.

In addition to receiving real-time data, stored medical data may also be received. Stored medical data may include x-ray data, ultrasound data, CAT scan data, test results or pharmacological data or any combination of the aforesaid.

From mobile device 117 (preferably taking the form of a laptop computer) the clinician makes a request via public network 116 to the data processing system 114. Within the third processing system 203 the request is decoded by subsystem 204 whereafter the input request is transmitted to the first processing system 201 by subsystem 205. In this initial state, output transmission is blocked therefore it is necessary for the calling clinician to invoke an authentication process.

Within processing system 202, subsystem 206 determines a password which is then transmitted to the clinicians mobile cellular telephone 111 (via the cellular telephone network) via subsystem 207. Thus, subsystem 207 converts the password generated by subsystem 206 into an SMS message that is sent to the clinician's mobile telephone 119; the number having been stored by the processing system when the clinician's account was established. Thus, it is not possible for anyone without an established account to receive information from the system and clinicians with established accounts must have their mobile phone to hand so that they can receive the SMS message.

Having received the SMS message identifying a "use only once" password (also known as a one-time password or OTP) the clinician enters this password using mobile device 117.

Within system 202, subsystem 208 checks whether an appropriate response has been made and when the question posed is answered in the affirmative, an enable signal is applied to system 203. With system 203 enabled, subsequent input received from the external clinician is supplied from the input subsystem 205 to the first processing system 201.

At the mobile device 117, the clinician makes a request for particular feed types from a particular patient to be supplied. For the purposes of this example, the clinician requests data from patient John Jones consisting of his current blood pressure, heart rate and heart trace.

In response to the request being received at processing system 201, subsystem 209 selects data feeds or sets from the local area network 111. These data sets are native to the particular device generating the data, in this example device 106 producing an ECG trace. The data is transmitted in its native form because this would facilitate the attachment of a similar device to 106 to the network such that the nature of the data would be meaningful and a print out could be obtained from such a similar device. Thus, all of the data transmitted within the network is native to the particular device generating the data.

Consequently, subsystem 210 processes the native data to produce an image and the image produced by subsystem 210 is converted into a generic video signal by subsystem 211. Thus, it is possible for this generic video signal to be displayed at a mobile device, such as mobile device 117 with minimal additional processing. Furthermore, data sets from several diverse equipment types may be combined into a single generic video signal such that several data sets relating to a particular patient may be displayed simultaneously at the remote mobile device. Within such an environment, the mobile device 117 may be considered as a "thin client" with the bulk of the processing being performed by the data processing system 115.

Having produced the generic video signal, this data is supplied to the third processing system 203 where subsystem 211 encrypts the data (using a conventional encryption technique such as SSL) and thereafter transmits the data via public network 116 to the requesting mobile device 117.

### Figure 3

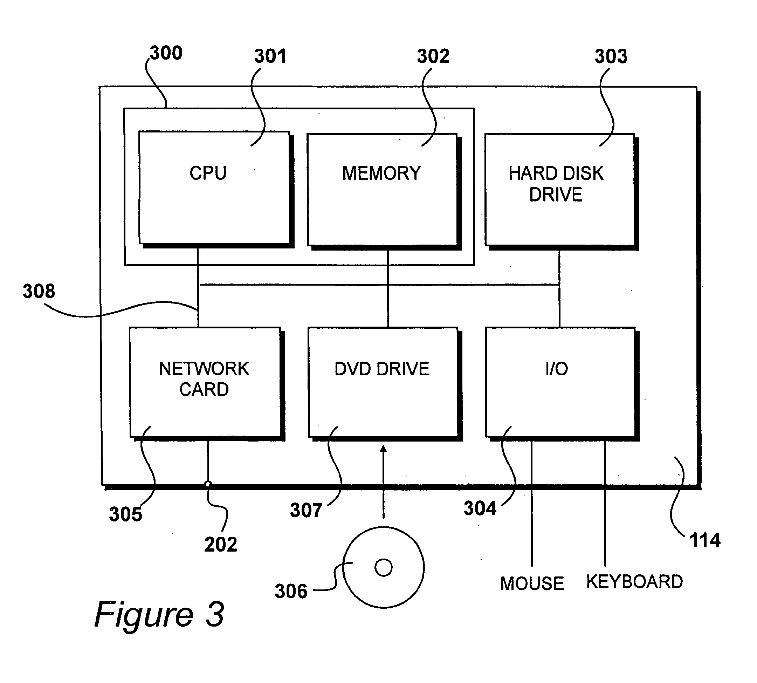

An example of components contained within data processing system 114 is shown in Figure 3. A core processing environment 300 includes a central processing unit 301 and randomly accessible memory 302, the latter being provided for the storage of programs and operational data executed by the central processing unit 301.

Storage for programs and operational data is also provided by a hard disk drive 303, although alternative forms of storage could be provided in alternative embodiments. An input/output interface 304 is provided for receiving input commands from a mouse and keyboard and for providing output to a display monitor. A network card 305 provides for connectivity to a network and new programs and data may be loaded from portable storage devices, such as disc 306 by an appropriate DVD drive 307. The components communicate via a system bus 308.

Operational functionality is provided by the core processing environment 300 implemented by programs held in memory 302 being executed by central processing unit 301. This usually involves the provision of an operating system with applications executing within the environment established by said operating system. However, in accordance with the present invention, three distinct processing environments are required which, in an alternative embodiment, could be provided by the establishment of three separate hardware platforms. However, in the preferred embodiment the processing systems are established by executing three separate operating systems upon the system hardware each co-ordinated by a hypervisor program.

### Figure 4

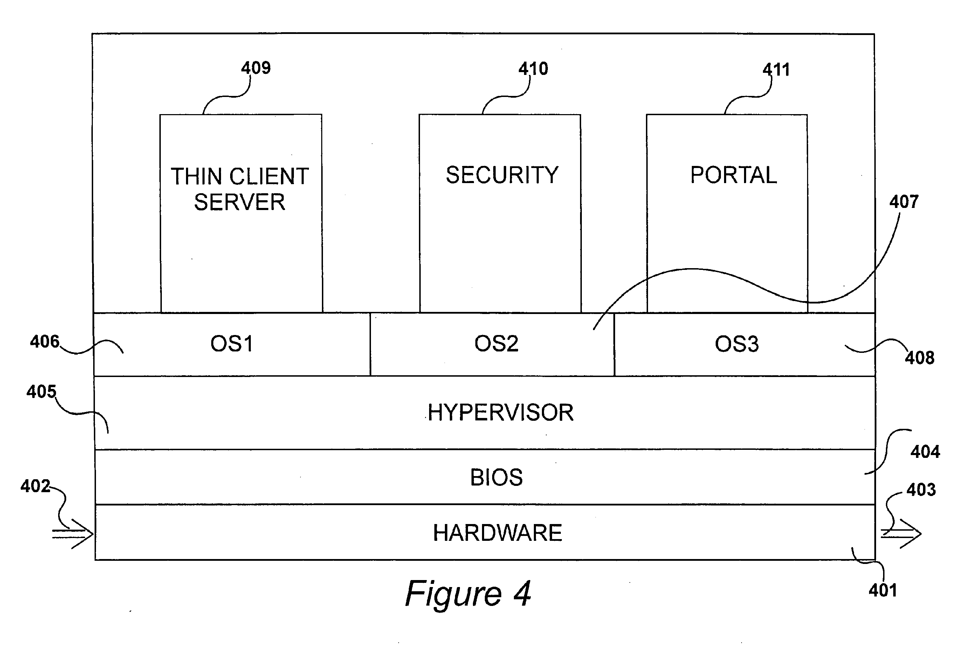

As illustrated in Figure 4, the functionality of the core processing environment 300 is shown as a hierarchy in which hardware 401 is arranged to receive input signals 402 and to supply output signals 403. Communication with the hardware is facilitated by a basic input/output system (BIOS) 404 which in conventional configurations would then be in direct communication with an operating system. However, in this embodiment, a hypervisor 405 is installed such as VMware ESX provided by Vmware Inc, Palo Alto, Califormia, 3401 Hillview Avenue, Palo Alto, CA, 94304, USA. The hypervisor software 405 allows separate installations of individual operating systems. In the present embodiment a first operating system 406 is installed, along with a second operating system 407 and a third operating system 408.

First operating system 406 provides a thin client server as represented at 409. Second operating system 407 provides security facilities as illustrated at 410. A third operating system 408 provides portal functionality as illustrated at 411.

### Figure 5

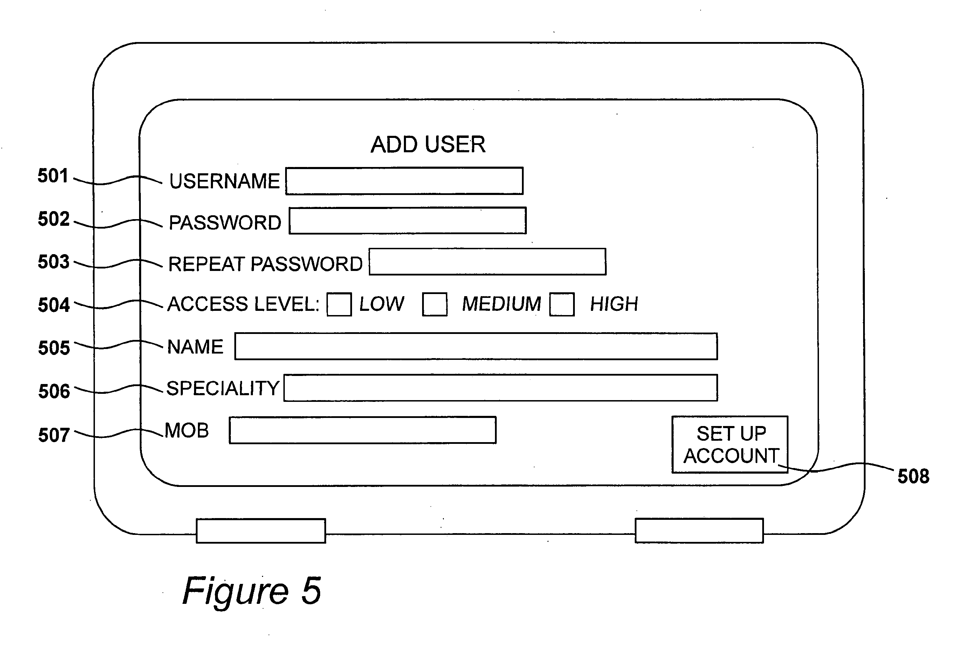

An example of a display shown in order to add a user (clinician) is shown in Figure 5. Text boxes are provided to enable a user's details to be added using a keyboard or similar input device. At 501 a box is provided for a username to be entered. This acts as a unique identifier. A password is identified at 502 and repeated at 503 for confirmation. At 504 an access level is defined. In this embodiment, different levels of access can be enabled for different users. Thus, for example, a senior member of staff would have a high access level and be able to access a larger amount of medical information than a more junior member of staff who may be assigned a low or medium access level. In alternative embodiments, many more access levels could be defined or the system could be configured such that members of staff only have access to patients with whom they are dealing directly. Alternatively, all members of staff registered on the system could have the same access level.

Further user details are entered such as name at 505, speciality at 506 and Mobile Telephone Number (MOB)at 507.

Once information has been entered a button 508 is pressed in order to set up the account.

### Figure 6

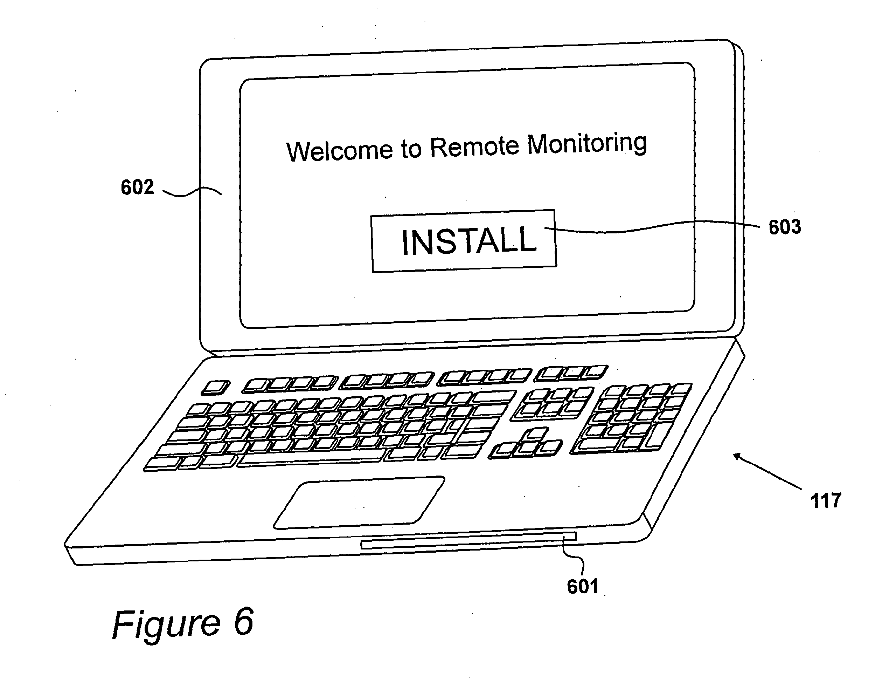

A mobile device 117 is shown in Figure 6 wherein the remote monitoring facility is being installed.

In this embodiment, a CD or DVD is inserted into the appropriate drive 601. The disc contains an application which is loaded onto mobile device 117 in order to allow information to be received relating to real-time medical data. Screen 602 is seen displaying text indicating that a disk is being inserted and providing a button 603 to be pressed in order to install the application. In alternative embodiments, an application may be for example downloaded via network connection or other means therefore such an installation may not be required.

### Figure 7

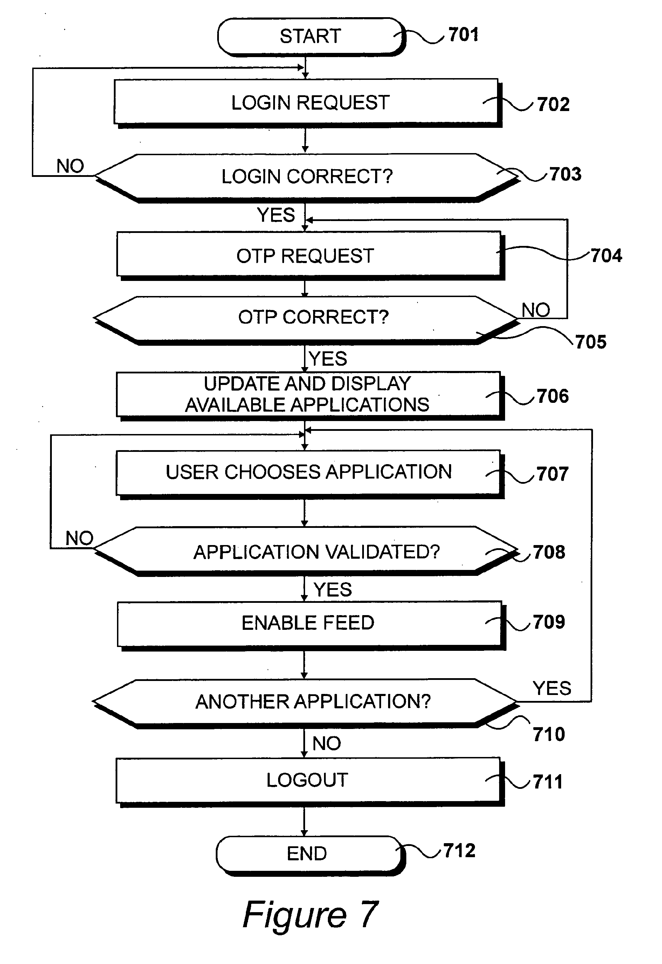

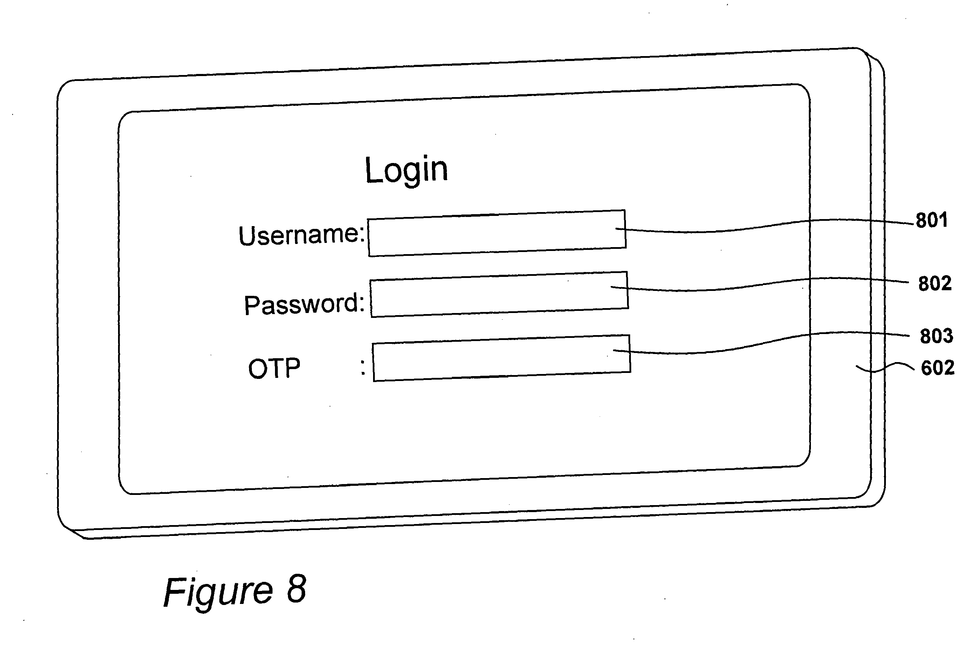

An overview of procedures which take place when a request is received from a clinician to receive data is shown in Figure 7. A session starts at 701 and at 702 a login request is received. An example of a screen used to input a login request is shown in Figure 8.

At step 703 a question is asked as to whether the login details are correct. If this question is answered in the negative then control passes back to step 702 at which an error message is displayed. If the question asked at step 703 is answered in the affirmative indicating that login details are correct then control passes to step 704. At step 704, the user is prompted to enter a one time password (OTP) sent by SMS message. At step 705 a question is asked as to whether the OTP is correct. If the question asked at step 705 is answered in the negative then control passes back to step 704. If the question asked at step 705 is answered in the affirmative indicating that the OTP is correct then control, passes to step 706. At step 706 the user is provided a newly updated menu of applications from which they may choose. At step 707 a user chooses an application and control passes to step 708 where the application is validated. If the question asked at step 708 is answered in the negative then control passes back to step 707. If the question asked at step 708 is answered in the affirmative indicating that validation was successful the control passes to step 709 where the application feed is enabled. At step 710 if another application is required control passes back to step 707. If no further applications are required, the user can logout of the service at step 711. Procedures then end at step 712.

### Figure 8

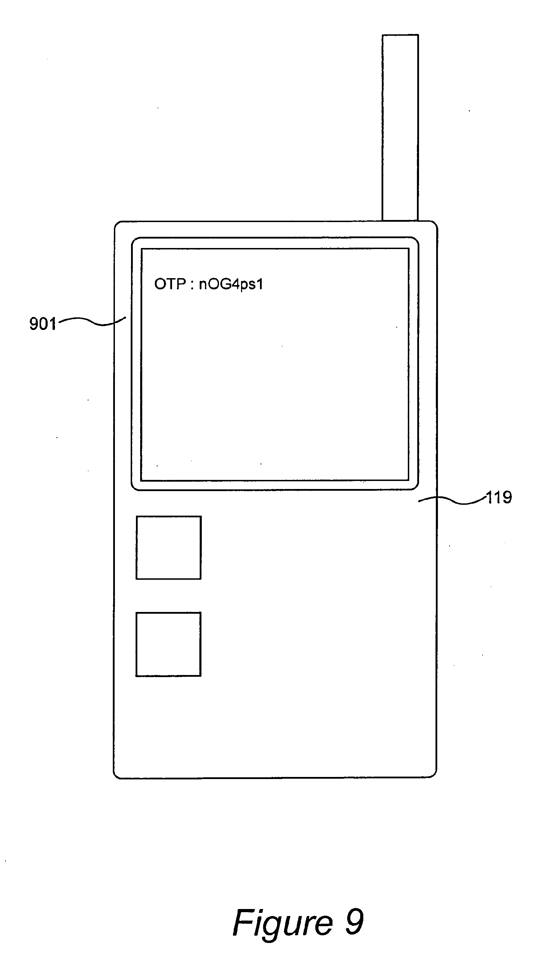

An example of a display shown on screen 602 at step 702 is shown in Figure 8. A box is provided at 801 for a username to be entered and a further box 802 for a password to be entered. In addition, a third box 803 is provided which requests an OTP (one-time password). The one-time password is, in this embodiment, provided on a per-session basis and supplied to the clinician via an alternative channel. An example of an alternative channel is shown in Figure 9, in the form of a mobile cellular telephone and the one-time password being received as a text message (SMS). Further alternatives include a radio pager or security tokens etc.

### Figure 9

An example of an alternative channel for receipt of a one-time password by a clinician is shown in Figure 9. In this example, mobile phone 119 has received the one-time password as shown at 901.

### Figure 10

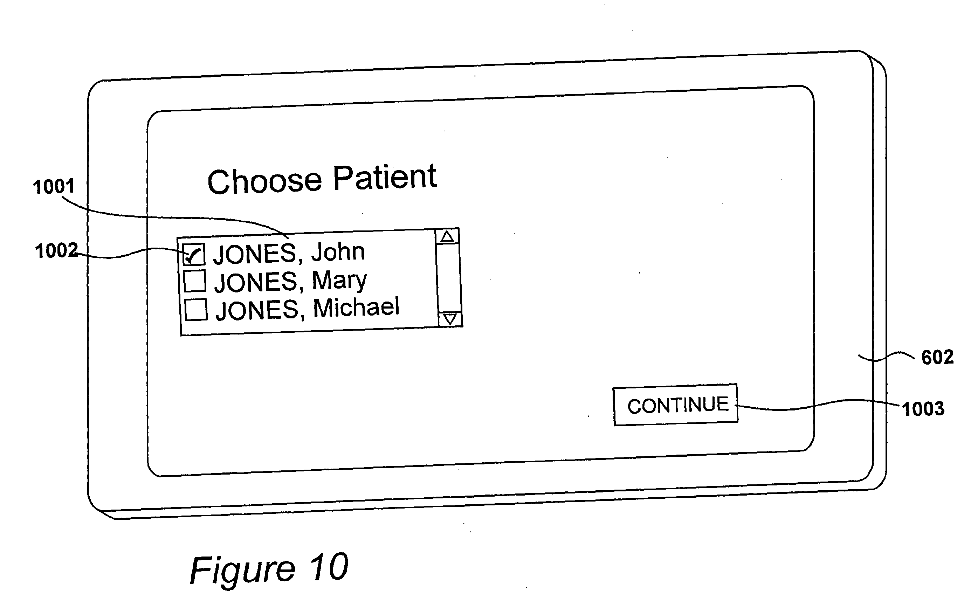

A display shown at step 704 on screen 602 is illustrated in Figure 10. A list of patients is provided at 1001 and tick boxes are provided to allow you to select a patient. In this example, tick box 1002 has been selected such that a clinician has chosen to view data relating to John Jones. Once a patient or patients has been selected in this way, a continue button is provided at 1003 to allow the clinician to proceed to the next stage.

### Figure 11

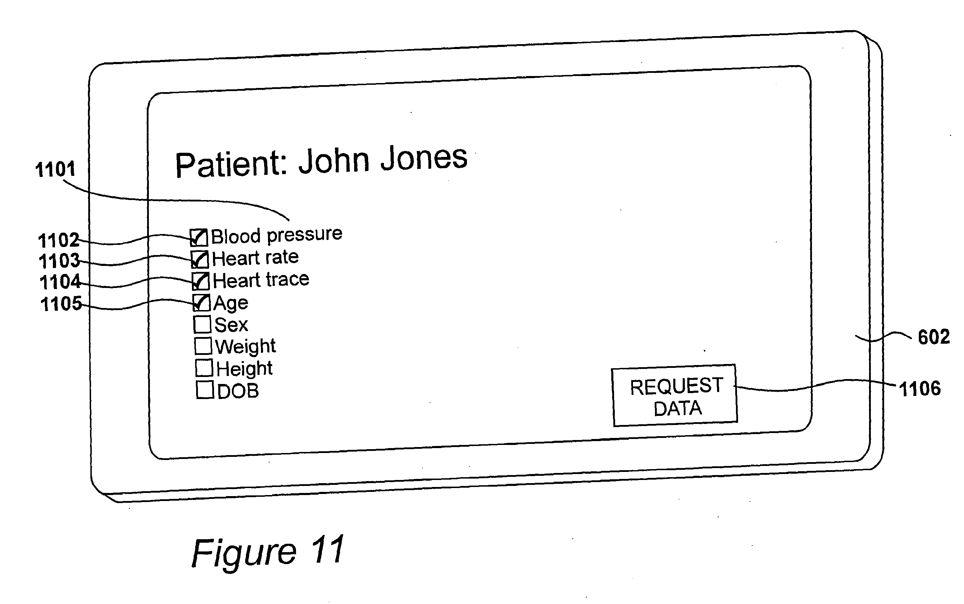

An example of a screen displayed on 602 at step 706 is shown in Figure 11. At this step, a clinician is prompted to choose which medical devices they wish to view and medical information from. A list is provided at 1101 and in this example tick boxes 1102, 1103, 1104 and 1105 have been selected. This indicates that the clinician wishes to view data relating to blood pressure, heart rate, heart trace and age of the patient in question. Once appropriate tick boxes have been selected, a button is provided at 1106 to request the data selected a button is provided at 1106 to request the data selected.

### Figure 12

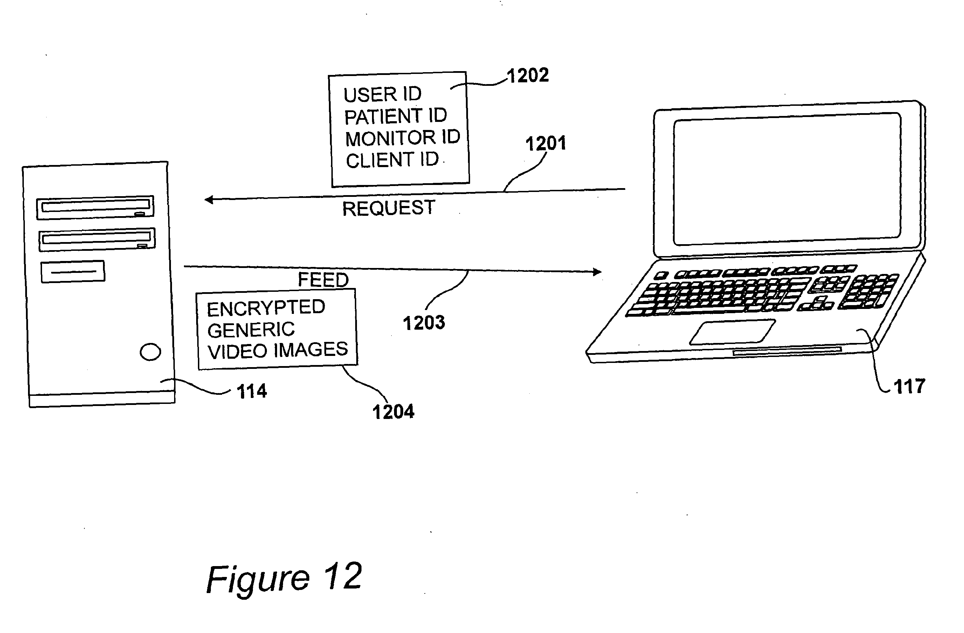

Interactions between mobile device (laptop) 117 and processing system 114 are illustrated in Figure 12. A request is sent from laptop 117 to processing system 114 as represented by arrow 1201. This request includes information for identifying the system it requested. In this example, a user ID, patient ID and monitor ID are supplied as illustrated at 1202. Once the request has been received in processing system 114 procedures in accordance with Figure 2 take place and, assuming successful authentication a feed is supplied back to laptop 117 as shown at 1203. The feed comprises encrypted generic video images as illustrated at 1204.

### Figure 13

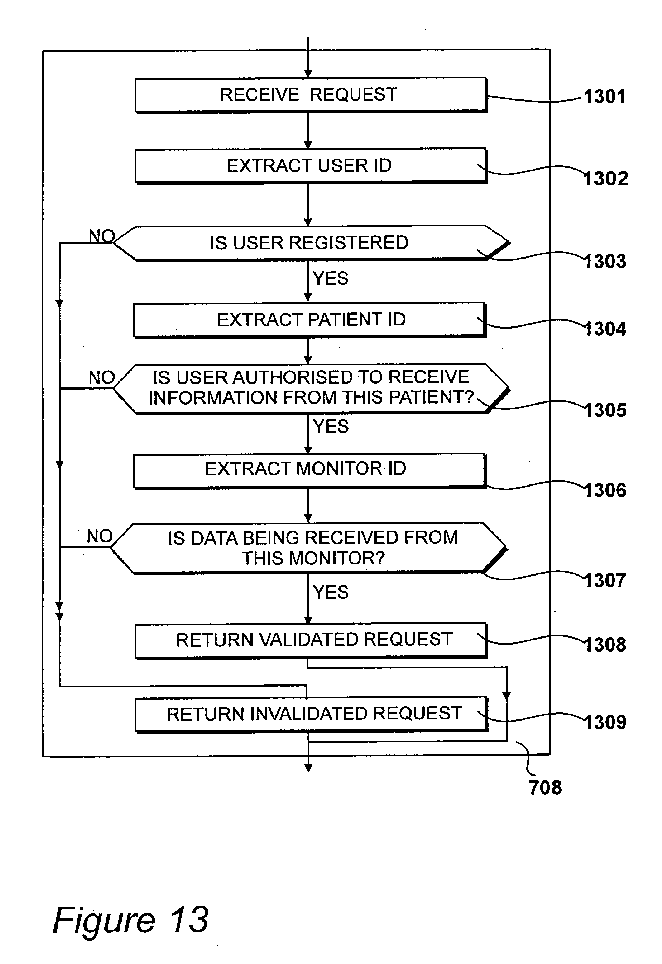

An expansion of step 708 in which a request is processed is shown in Figure 13. At step 1301, a request is received and the user ID is extracted from the request at step 1302. The user ID identifies the specific clinician who is requesting information.

At step 1303 a question is asked as to whether the user is registered. If this question is answered in the negative then an invalidated request is returned at 1309. If the question answered at step 1303 is answered in the affirmative indicating that the user (clinician) is registered then control passes to step 1304. At step 1304, the patient ID is extracted. This identifies which specific patient information is required relating to. A question is asked at step 1305 as to whether the user is authorised to receive information relating to this patient. If this question is answered in the negative an invalidated request is returned at step 1309. If this question is answered in the affirmative indicating that the clinician is authorised to receive data from this patient then control passes to step 1306.

At step 1306, a monitor ID is extracted. The monitor ID identifies the specific apparatus generating medical data (such as a heart rate monitor) from which information is requested. At step 1307 a question is asked as to whether data is being received from this monitor. If this question is answered in the negative then an invalidated request is returned at 1309. If the question asked at 1307 is answered in the affirmative indicating data is being received from this monitor then control passes to step 1308. At step 1308 a validated request is returned.

### Figure 14

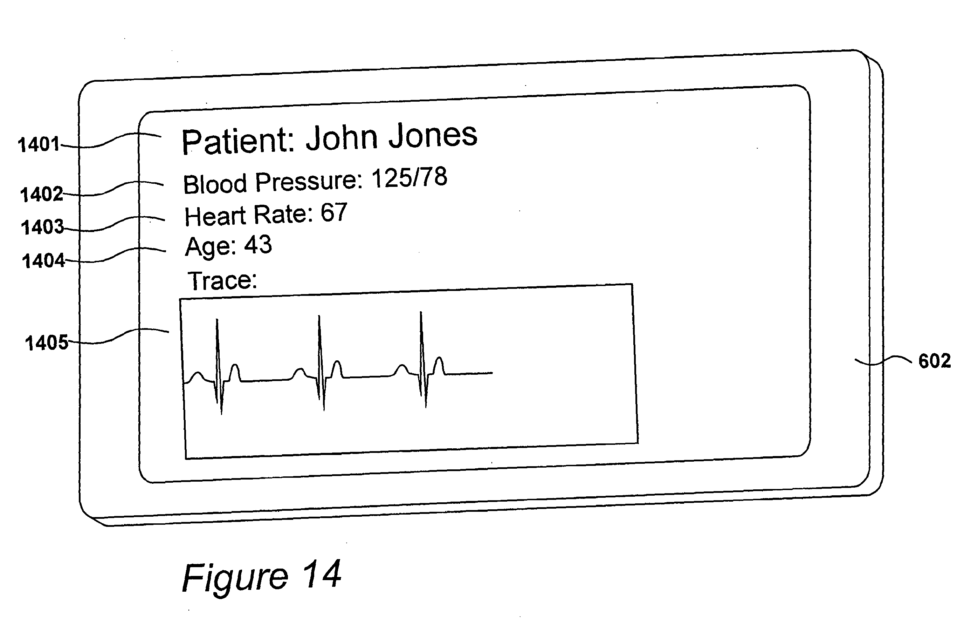

An example of medical data being displayed is shown in Figure 14. Screen 602 is shown with a display which includes information identifying a patient at 1401, the patient's blood pressure at 1402, the patient's heart rate at 1403, their age at 1404 and a trace of the heart at 1405. This information corresponds with that requested by the clinician as described with reference to Figure 11.

The information displayed as shown in Figure 14 has been generated in accordance with procedures described with reference to Figure 2. Therefore, the information displayed is a generic video image created from the originating data feed. In accordance with individual configuration, the generic video is encrypted before transmitting in order to enhance security.

## Claims

1. Apparatus for conveying real-time medical data received from patient-connected terminals within a hospital to clinicians, **characterised in that**:
said clinicians receive data from the apparatus via mobile devices located outside a hospital; and
said apparatus is located at said hospital collecting a plurality of data sets from a plurality of patients, wherein the apparatus comprises:
a first processing system for receiving native medical data from a hospital internal network and for converting said data into generic video images;
a second processing system for authenticating requests from mobile devices to facilitate or block external communication; and
a third processing system for receiving input commands from said mobile devices, encrypting said generic video images to produce encrypted video images and for transmitting said encrypted video images to said mobile devices.

2. Apparatus according to claim 1, **characterised in that** said patient-connected terminals produce electrocardiograph (ECG) trace data, blood pressure data and fluid flow data in real-time.

3. Apparatus according to claim 1 or claim 2, **characterised in that** said processing device receives stored medical data in addition to said real-time data.

4. Apparatus according to claim 3, **characterised in that** said stored medical data is x-ray data, ultrasound data, CAT scan data, test results or pharmacological data or any combination of the aforesaid.

5. Apparatus according to any of claims 1 to 4, **characterised in that** said processing systems are supported on a shared hardware platform that runs each system on a dedicated operating system.

6. Apparatus according to claim 5, **characterised in that** said operating systems communicate with a shared BIOS via a hypervisor platform.

7. Apparatus according to any of claims 1 to 6, **characterised in that** input commands received by said third processing system are conveyed to said first processing system, in response to which real-time medical data is selected, processed and supplied to the third processing system as generic video data.

8. Apparatus according to any of claims 1 to 7, **characterised in that** said second processing device generates a one-time password on a per-session basis which is supplied to said clinician via an alternative channel.

9. Apparatus according to claim 8, **characterised in that** said one-time password is supplied to the clinician as a text message to a mobile cellular telephone in the possession of said clinician.

10. Apparatus according to any of claims 1 to 9, **characterised in that** the apparatus is provided with multiplexing capabilities such that a plurality of data sets for respective patients along with patient identifying data is supplied to a clinician as a generic video image.

11. Apparatus according to claim 10, **characterised in that** a plurality of graphic images, each displaying multiple data sets, are supplied to respective ones of a plurality of clinicians.

12. A method of conveying real-time medical data received from patient-connected terminals within a hospital to clinicians, **characterised in that** a plurality of data sets from a plurality of patients are collected within a hospital, and supplied to clinicians via mobile devices located outside a hospital, comprising the steps of:
receiving native medical data from a hospital internal network;
converting said data into generic video images;
authenticating requests from mobile devices to facilitate or block external communication;
encrypting said generic video images to produce encrypted video images;
transmitting said encrypted video images to said mobile devices.
